# EUROPEAN PATENT APPLICATION

(11) **EP 4 603 112 A1**
(43) Date of publication of application: **20.08.2025**
(21) Application number: 24157736.0
(22) Date of filing: 15.02.2024
(51) Int. Cl.: A61L 27/26, A61L 27/50

(54) **METHOD AND COMPOSITION FOR INTRAOCULAR LENSES WITH ADJUSTABLE UNFOLDING TIME**

(71) Applicant: ACTIOL GmbH, 35287 Amöneburg (DE)
(72) Inventor: KIM, Hee-Cheol, 35039 Marburg (DE)
(74) Representative: Fuchs Patentanwälte Partnerschaft mbB

(57) **Abstract**

Disclosed are a method and compositions for the production of polymers for intraocular lenses which have an adjustable unfolding time. The unfolding time is adjusted by varying the ratio of two crosslinkers having different chain lengths. Other properties of the intraocular lenses remain essentially unaltered when adjusting the unfolding time.

## Description

The present disclosure relates to a method and composition for the production of ophthalmic medical aids such as intraocular lenses and preforms thereof which have an adjustable unfolding time. The unfolding time is adjusted by varying the ratio of two crosslinkers having different chain lengths. Other properties of the intraocular lenses remain essentially unaltered when adjusting the unfolding time.

### Background

Intraocular lenses (IOLs) are implanted into the eye by an eye surgeon using so-called "shooters". A shooter is basically a syringe with a relatively thick cannula which receives the folded or rolled up IOL and can be inserted through a small cut into the capsular bag. There, the IOL is pushed out of the cannula and placed in the capsular bag. This is done in operating rooms typically having a room temperature of 16 °C - 20 °C.

After the IOL is placed in the capsular bag, the folded or rolled up IOL warms up to the internal temperature in the capsular bag, which is approximately 34 °C, and unfolds. This unfolding process requires a certain unfolding time. The unfolding time should not be too short because this could damage the capsular bag. However, it should also not be too long because only after complete unfolding of the IOL, the eye surgeon can assess the correct position of the IOL and correct it if necessary. It depends on the assessment and preferences of the eye surgeon which time span for the complete unfolding of the IOL seems appropriate. However, at present the lOLs available on the market are not sold with an indication of unfolding times. Instead, it remains a matter of experience of the eye surgeon to estimate these times.

Moreover, standard polymeric materials for ophthalmology have a specific unfolding time that is typical for the respective material. For this reason, at present the lOLs offered are only available with a single unfolding time. Hence, if an eye surgeon is not satisfied with the unfolding time of a certain IOL, the only option is to change the type of IOL to another polymeric material or composition. There is no IOL available which offers different unfolding times while keeping its other relevant properties unaltered.

Therefore, there is a need for lOLs with a complete deployment within a certain time window and also for lOLs which can be adjusted in their unfolding time.

### Summary of this disclosure

In a first aspect, this disclosure relates to a method for making an intraocular lens or a preform thereof comprising
- providing a polymerizable composition comprising
   + at least one polymerizable monomer, oligomer, or polymer,
   + at least a first and a second crosslinker, wherein the first and second crosslinkers have a generic formula A1 - (B)_{q} - A2, with A1, A2 = crosslinkable group and B = backbone group of C, O, Si, and/or S atoms, and wherein first and second crosslinkers differ in a chain length q of the molecules by at least 1 and optionally at most 40, and
   + at least one initiator,
- selecting a molar ratio of first to second crosslinker, and
- polymerizing the composition to form the intraocular lens or a preform thereof.

First and second crosslinkers may differ in the chain length q of the molecules by at least 1 or optionally by at least 2 or at least 3 or at least 4 or at least 5 or at least 6 or at least 7 or at least 8 or at least 9 or at least 10. First and second crosslinkers may optionally differ in the chain length q of the molecules by at most 40 or at most 39 or at most 38 or at most 37 or at most 36 or at most 35 or at most 34 or at most 33 or at most 32 or at most 31 or at most 30. First and second crosslinkers may optionally differ in the chain length q of the molecules in a range of from 1 to 40 or from 2 to 39 or from 3 to 38 or from 4 to 37 or from 5 to 36 or from 6 to 35 or from 7 to 34 or from 8 to 33 or from 9 to 32 or from 10 to 31 or from 10 to 30.

In a second aspect, this disclosure relates to a composition for intraocular lenses or preforms thereof comprising
- at least one polymerizable monomer, oligomer, or polymer,
- at least a first and a second crosslinker, and
- at least one initiator,
wherein the first and second crosslinkers have a generic formula A1 - (B)_{q} - A2, with A1, A2 = crosslinkable group and B = backbone group of C, O, Si, and/or S atoms, and differ in a chain length q of the molecules by at least 1 and optionally at most 40 and wherein an intraocular lens or preform thereof produced from this composition by polymerization has an unfolding time which is adjustable by selecting a molar ratio of first to second crosslinker.

In a third aspect, this disclosure relates to an article in the form of an ophthalmic medical aid, in particular an intraocular lens or a preform thereof, comprising a polymerized product obtained by polymerizing a composition according to the second aspect, and/or by performing the method according to the first aspect.

### Details of this disclosure

The details of this disclosure relate to the aspects described in the summary of disclosure. Any of the features of the embodiments described hereinafter may relate to the method for making an intraocular lens or a preform thereof as well as the composition for intraocular lenses or preforms thereof and the article in the form of an ophthalmic medical aid.

### Generic formula

In abovementioned generic formula A1 - (B)_{q} - A2, the crosslinkable groups A1 and A2 may be the same or different, as required by the applied crosslinking reaction. Further, the backbone groups B are characterized in this context only by the presence of a chain of the respective substituted or unsubstituted C, O, Si, and/or S atoms as linkers between the crosslinkable groups. As opposed to a repeating unit of a polymer, each backbone group B may be a different one of the four atoms and may also be substituted differently. For example, with q = 2, the resulting group -B-B- may consist of a -CH₂-O- group (a substituted C and an unsubstituted O) as well as a -CH₂-CH₂- group (two identically substituted C) or of a -CH₂-C(CH₃)H- group (two differently substituted C). The relevant feature is the space between the crosslinkable groups created by the molecular backbone chain of B atoms (-C-O- in the first case and -C-C- in the latter two cases). If the backbone group (B)_{q} comprises a ring structure, only those atoms of the ring structure will be counted for q which form the shortest distance in the chain between A1 and A2. For example, if the chain contains in a 1,3-substitution pattern a benzene or cyclohexyl ring, only the three C's number 1, 2, and 3 will be counted for q but not the five C's number 1, 3, 4, 5, and 6. In other words, "q" is the number of C, O, Si, and/or Si of the shortest covalent chain linking A1 and A2, thereby providing a good measure of distance between the crosslinkable groups.

The crosslinkable groups A1 and A2 may optionally be selected independently of each other from the group comprising acrylate group, methacrylate group, vinyl group, alkyne group, amine group, aldehyde group, epoxy group, carbonic acid group, and hydroxy group, in particular for radical or ionic polymerization processes. The crosslinkable groups do not necessarily have to be copolymerizable with the polymerizable monomer, oligomer, or polymer of the composition but may also react in a polymer analogous reaction with respective side groups of the polymer chains for crosslinking them.

The inventors have surprisingly found that by varying the amount of a single crosslinker the unfolding time cannot be adjusted. While one might expect that the amount of crosslinker has an influence on the density of the created polymer network and, therefore, also on the hardness of the produced IOL which in turn has an impact on the unfolding time, the results of respective tests have shown that the unfolding time remains essentially constant. However, when using a pair of two different crosslinkers with differing chain lengths, the unfolding time can be freely and reliably adjusted between the respective unfolding times of the IOLs produced with the single crosslinkers by varying their ratio in the pair. For achieving a different range, it suffices to select a different pair of crosslinkers.

In optional embodiments, the spacer between the crosslinkable groups A1 and A2 formed by the backbone groups (B)_{q} may be in the form of repeating units, such as ethylene glycol units, propylene glycol units, or the like. The two crosslinkers may, hence, differ in the number of repeating units.

It may be advantageous to use two crosslinkers having an identical repeating unit because their optical properties remain very similar when varying their number.

In optional embodiments, following a general formula A1 - spacer₁ - (B)_{q} - spacer₂ -A2, two crosslinkers having identical crosslinkable groups A1 and A2 and an identical middle part of the backbone group but different spacers between them may be used.

In optional embodiments, when defining the two crosslinkers with a general formula

A1_{B} - spacer_{B1} - (B), - spacer_{B2} -A2_{B}

and

A1_{C} - spacerc, - (C)ₛ - spacer_{C2} -A2_{C},

with A1_{B,C}, A2_{B,C} = crosslinkable group, B, C = backbone group of C, O, Si, and/or S atoms, and spacer_{B1, C1}, spacer_{B2, C2} = 1-10 repeating units,
wherein first and second crosslinkers differ
   i) in a chain length r and s of the molecules by at least 1 and optionally at most 40 and/or
   ii) in a number of repeating units in spacer_{B1, C1} and/or spacer_{B2, C2},
and wherein spacer_{B1} ≠ spacer_{C1},
one or more, optionally all, of the following definitions may apply:
   a) A1_{B} = A2_{B} = A1c = A2c,
   b) (B)ᵣ = (C)ₛ,
   c) spacer_{B1} = spacer_{B2} and spacer_{C1} = spacer_{C2}.

An advantageous combination may use A1_{B} = A2_{B} = A1_{C} = A2_{C}. In a more advantageous combination, also B = C is used. In a most advantageous combination, spacer_{B1} = spacer_{B2} and spacer_{C1} = spacer_{C2}. In all of these combinations, always spacer_{B1} ≠ spacer_{C1} applies.

### Unfolding time

The unfolding time of an IOL or precursor thereof is defined as the timespan determined at a temperature of 34 °C between the folded or rolled IOL or precursor leaving the cannula of a shooter and reaching a fully unfolded or unrolled state characterized by no change of more than 1° within 1 min. It can be measured using a video recording as follows.
- An IOL precursor (also commonly called button) of 2 mm thickness and 12 mm diameter is rolled up and put at a room temperature of 20 °C - 25 °C into a pushout device comprising a PTFE tube of 10 mm diameter and a PTFE plunger.
- Then the button is allowed to unfold in the PTFE tube for 45 s in order to have a defined degree of folding at the start of the measurement for all samples.
- Thereafter, the pushout device is positioned above a heating plate which is tempered at 34 °C in the field of view of a camera recording a video of the unfolding procedure and the button is pushed out by the plunger onto the heating plate. For example, a USB camera Owl Mirazoom MZ902 can be used for this purpose.
- The time elapsed until complete unfolding is determined by evaluating frame shots of the video. Complete unfolding is considered to be achieved when no change of more than 1° within 1 min occurs.
- This procedure is executed with four buttons and mean value and standard deviation are determined using a data analysis software such as QTI Plot.

The same procedure can be used for measuring an IOL instead of the button.

In embodiments, the method may comprise
- providing a first polymerizable composition comprising
   + at least one polymerizable monomer, oligomer, or polymer,
   + at least a first and a second crosslinker, wherein the first and second crosslinkers have a generic formula A1 - (B)_{q} - A2, with A1, A2 = crosslinkable group and B = backbone group of C, O, Si, and/or S atoms, and wherein first and second crosslinkers differ in a chain length q of the molecules by at least 1 and optionally at most 40, and
   + at least one initiator,
- selecting a molar ratio R1 of first to second crosslinker, and
- polymerizing the first composition to form a first intraocular lens or a preform thereof; and
- providing a second polymerizable composition comprising
   + the polymerizable monomer, oligomer, or polymer; the first and second crosslinkers; and the initiator,
- selecting a molar ratio R2 of first to second crosslinker, and
- polymerizing the second composition to form a second intraocular lens or a preform thereof.

Here, the steps of providing the first and second polymerizable compositions may also be performed in temporally and/or spatially separated steps. Consequently, the consecutive steps of selecting the molar ratios R1 and R2 and polymerizing the first and second polymerizable compositions may likewise be performed in temporally and/or spatially separated steps. There is no need to produce the intraocular lenses or preforms thereof at the same time and place.

Also in these embodiments, first and second crosslinkers may differ in the chain length q of the molecules by at least 1 or optionally by at least 2 or at least 3 or at least 4 or at least 5 or at least 6 or at least 7 or at least 8 or at least 9 or at least 10. First and second crosslinker may optionally differ in the chain length q of the molecules by at most 40 or at most 39 or at most 38 or at most 37 or at most 36 or at most 35 or at most 34 or at most 33 or at most 32 or at most 31 or at most 30. First and second crosslinker may optionally differ in the chain length q of the molecules in a range of from 1 to 40 or from 2 to 39 or from 3 to 38 or from 4 to 37 or from 5 to 36 or from 6 to 35 or from 7 to 34 or from 8 to 33 or from 9 to 32 or from 10 to 31 or from 10 to 30.

With these embodiments of the method, it is possible to produce two variants of a specific intraocular lens or preform thereof which essentially only differ in their unfolding time.

In embodiments, the method may further comprise the steps of
- receiving a desired unfolding time for the intraocular lens, particularly from a medical practitioner, and
- selecting the molar ratio of first to second crosslinker such that the desired unfolding time is obtained.

It is a great advantage of the disclosed method that it will allow for a tailoring of the unfolding time to the preferences of a medical practitioner such as an eye surgeon. This means that not only a range of various unfolding times for a given IOL type can be offered but also a particular desired unfolding time can be produced on demand.

In embodiments, a total molar amount of crosslinker and/or a molar amount of initiator in the polymerizable composition may be held constant while selecting the ratios of first to second crosslinker, in particular the total molar amount of crosslinker and/or molar amount of initiator in the first and second polymerizable compositions may be essentially the same. For adjusting the unfolding time, it is not necessary to vary the total content of crosslinkers or initiator. It will suffice to select the ratio accordingly while the total amount may be kept constant. This has the advantage of a reduced impact on other important mechanical and optical properties of the IOL.

In embodiments, a ratio of the longest unfolding time to the shortest unfolding time at 34 °C may be at least 2:1, optionally at least 5 : 1 or at least 10 : 1, in particular a ratio of the longest unfolding time to the shortest unfolding time at 34 °C comparing the first and second intraocular lens or preform thereof may be at least 2:1, optionally at least 5 : 1 or at least 10 : 1. With the disclosed method it is possible to adjust the unfolding time over a quite large range.

In embodiments, the unfolding time at 34 °C may be adjustable in a range of from 15 s to 300 s and/or may be varied with the ratio of the two crosslinkers in a linear or exponential dependency, in particular the unfolding time at 34 °C comparing the first and second intraocular lens or preform thereof may be adjustable in a range of from 15 s to 300 s and/or may be varied with the ratio of the two crosslinkers in a linear or exponential dependency. By selecting particular crosslinkers, it is possible to have either a linear or exponential dependency of the unfolding time on their ratio. This may, for example, be useful in cases where less of one of the crosslinkers should be used in order to minimize its effect on the other properties of the produced IOL or if it is particularly expensive.

In embodiments, selecting the ratio of first to second crosslinker within a given composition changes one or more, optionally all, of the properties
- glass transition temperature T_{g},
- refractive index, determined at 589.3 nm and 20 °C,
- v (Abbe),
- absorption spectrum,
of the intraocular lens or the preform thereof by less than 10 %, optionally by less than 5 %, relative to the lowest one of the compared values.

When referring to "the absorption spectrum changes by less than 10 %, optionally by less than 5 %" this means that the integral of the absorption spectrum in the visible spectrum range of 400 nm to 800 nm differs by less than 10 % or less than 5 %, respectively. It is particularly advantageous if these mechanical and optical properties of the intraocular lens and especially the preform thereof remain almost constant. The most important reason is that the machining parameters will have to be readjusted for each different unfolding time if the optical properties of the material changes too much when adjusting the ratio of the crosslinkers. Furthermore, it will facilitate certification of the IOL and its preform as a medical device.

In embodiments, a Young's modulus determined according to DIN EN ISO 527-1:2019-12 and an International Rubber Hardness Degree IRHD determined according to DIN ISO 48-2:2021-02 of the intraocular lens or the preform thereof may be increased essentially proportional to the unfolding time.

In embodiments, the polymerizable composition may comprise a UV absorbing component and/or a blue light absorbing component. By incorporating UV and/or blue light absorbing components, an additional protection of the eye can be generated. It is possible to create a transmission spectrum of the IOL which resembles the naturally aged eye which above an age of about 50 almost completely blocks UV wavelengths below 400 nm and greatly reduces blue light wavelengths between 400 nm and 500 nm.

In embodiments, the first and second crosslinkers may be bifunctional acrylic or methacrylic compounds, optionally α,ω-substituted. These compounds show a good compatibility with the lens material and a fast reaction. If they are α,ω-substituted, the whole chain length can be used for forming the crosslinked network. Furthermore, there is less or no steric hindrance of the crosslinking reaction.

In embodiments, the first and second crosslinkers may be independently chosen from acrylic or methacrylic compounds having the formula 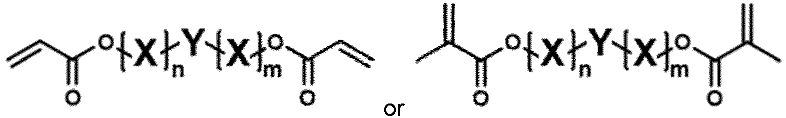
with n, m = 1-10; X = -CH₂-O-, -CH₂-CH₂-O-, -CH₂-CH₂-CH₂-O-, -S-O-, -S-S-O-, or -S-S-S-O-;
Y = bisphenol A, where A1 and A2 of the generic formula correspond to the acrylate or methacrylate group and (B)_{q} corresponds to [X]ₙ-Y-[X]ₘ,
or having the formula 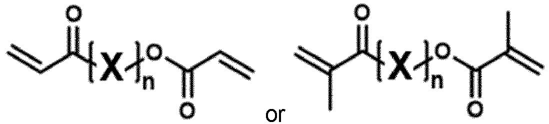
with n = 1-10; X = -CH₂-O-, -CH₂-CH₂-O-, -CH₂-CH₂-CH₂-O-, -S-O-, -S-S-O-, or -S-S-S-O-,
where A1 and A2 of the generic formula correspond to the acrylate or methacrylate group and (B)_{q} corresponds to [X]ₙ.

These specific crosslinkers can be used with most of the common lens materials and provide a wide variety of options for tailoring the unfolding times. Particularly, if both crosslinkers are chosen from these compounds, the created network will be very homogeneous due to the great similarity of the crosslinkers. Moreover, the most practically interesting unfolding times can be achieved with these compounds very easily and reproducibly. While the sulfur compounds effectively produce the same results as the carbon compounds regarding the unfolding times, they can be used to increase the refractive index of the IOL when compared to the respective carbon compounds.

In embodiments, the method may comprise the step of making two or more intraocular lenses or preforms thereof, wherein the two or more intraocular lenses or preforms thereof differ from each other in unfolding times, but not in the polymerizable monomers, oligomers, or polymers and/or initiators from which they are made. In other words, the method can be used to produce a certain type of IOL having a range of predetermined unfolding times. Hence, the medical practitioner is free to select a suitable or preferred type of IOL according to the needs of the patient and order it with an unfolding time according to his or her own preference or a specific condition of the patient's eye.

In embodiments, the polymerizable monomer, oligomer, or polymer may comprise (meth)acrylates, acrylamides and/or alkylene glycols and/or may be selected from the group comprising methyl methacrylate (MMA), 2-hydroxyethyl methacrylate (HEMA), 2-hydroxyethyl acrylate (HEA), butyl acrylate (BA), lauryl acrylate (LA), ethoxyethyl methacrylate (EOEMA), methacrylic acid (MAA), acrylamide (AA), ethylene glycol, propylene glycol, poly(ethylene glycol) phenyl ether acrylate (PEGPEA), and 2-phenylphenoxyethyl acrylate (OPPEA). These components are well suited for the production of lOL's and have proven themselves to be very well adjustable in unfolding time especially when used in combination with the abovementioned crosslinkers.

In embodiments, the article may have an unfolding time at 34 °C in a range of from 15 s to 300 s. These unfolding times at the temperature within the capsular bag can cover the range which is of particular interest for the eye surgeon.

### Brief description of the Figures

- **Figure 1**: shows the absorption spectra of EGDMA, TEGDMA, BPA-DMA, and BPAEO-DMA in the range of 200 nm - 800 nm.
- **Figure 2**: shows the absorption spectra of EGDMA, TEGDMA, BPA-DMA, and BPAEO-DMA in the range of 200 nm - 400 nm.
- **Figure 3**: shows a diagram of the unfolding time vs. the amount of the longer crosslinker of the system EGDMA + TEGDMA.
- **Figure 4**: shows a diagram of the unfolding time vs. the amount of the longer crosslinker of the system BPA-DMA + BPAEO-DMA.
- **Figure 5**: shows a diagram of the unfolding time vs. the amount of the crosslinker EGDMA.

### Examples

For demonstration of the effects achievable with the present disclosure, a model IOL composition has been chosen and tested with different crosslinker combinations. For comparison, the same model IOL composition has been tested with different amounts of the single crosslinkers also used in the combinations. The model IOL composition consisted of 95 mol-% of ethylene glycol phenyl ether acrylate (EDPEA) and 5 mol-% of the crosslinkers. This composition has been photopolymerized using 0.29 mol-% camphorquinone (CQ) and 0.25 mol-% ethyl-4-(dime-thylamino)benzoate (EDMAB) each relative to the total amount of the polymerizable composition.

Further, the buttons produced this way have been examined regarding their mechanical and optical properties. The glass transition temperature T_{g} has been determined according to DIN 51007:2019-04 with differential scanning calorimetry (DSC) at a rate of 10 K/min. The refractive index RI and the Abbe number v (Abbe) have been determined according to DIN EN ISO 489:2022-06 at 589.3 nm and 20 °C. The International Rubber Hardness Degree IRHD-M has been determined according to DIN ISO 48-2:2021-02.

The following **Table 1** lists the materials used in the examples.

**Table 1**

| **Substance** | **Structural formula** | **Sum formula** | **Molecular mass [g/mol]** |
|---|---|---|---|
| Ethylene glycol phenyl ether acrylate (EGPEA) | 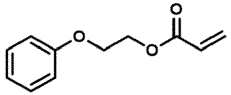 | C11H12O3 | 192.21 |
| Ethylene glycol dimethacrylate (EGDMA) | 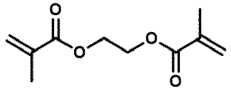 | C10H14O4 | 198.22 |
| Tetraethylene glycol dimethacrylate (TEGDMA) | 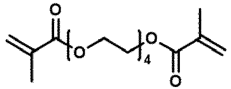 | C16H26O7 | 330.38 |
| Bisphenol A dimethacry-late (BPA-DMA) | 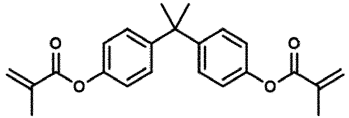 | C23H24O4 | 364.44 |
| Bisphenol A ethoxylate dimethacrylate (BPAEO-DMA) | 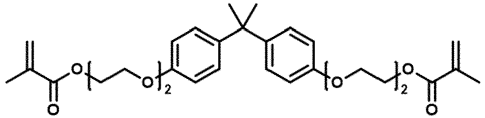 | C31H40O8 | 540.65 |

In these example materials, the equivalents of the generic formula A1 - (B)_{q} - A2 are as follows:
- For EGDMA, A1 and A2 are methacrylate groups 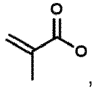 the chain of B's is C-C, and q is 2.
- For TEGDMA, A1 and A2 are methacrylate groups 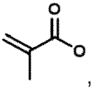 the chain of B's is C-C-O-C-C-O-C-C-O-C-C, and q is 11.
- For BPA-DMA, A1 and A2 are methacrylate groups 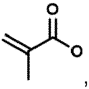 the chain of B's is C-C=C-C-C-C=C-C=C, and q is 9.
- For BPAEO-DMA, A1 and A2 are methacrylate groups 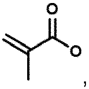 the chain of B's is (C-C-O-C-C-O)-C-C=C-C-C-C=C-C=C-(O-C-C-O-C-C), and q is 21.

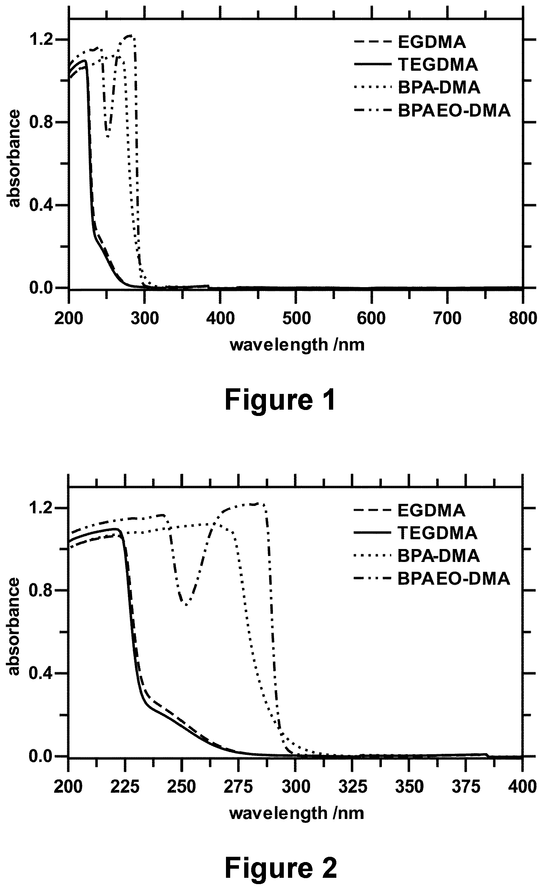

**Figure 1** shows the UV/vis absorption spectra of EGDMA, TEGDMA, BPA-DMA, and BPAEO-DMA measured at a concentration of 1 mmol/l in acetonitrile in the range of 200 nm - 800 nm. **Figure 2** shows an enlarged view of the measurement of Figure 1 in the range of 200 nm - 400 nm. As can be seen in these Figures, the absorption and transmission of the crosslinkers are almost the same within the pairs EGDMA / TEGDMA and BPA-DMA / BPAEO-DMA, especially for the first pair. This means that, when varying the ratio of these crosslinkers within these pairs while using them for adjusting the unfolding time of an IOL, also the absorption and transmission of the produced IOL will essentially remain the same. As mentioned above, this is particularly advantageous for certification of the IOL as a medical product.

### Example A: EGDMA + TEGDMA

The first system of crosslinkers which has been tested used ethylene glycol dimethacrylate (EGDMA) and tetraethylene glycol dimethacrylate (TEGDMA). Hence, the two crosslinkers differ only in the number of ethylene glycol units (1 vs. 4). The ratio of the two crosslinkers which are forming a total amount of 5 mol-% in the composition has been altered in 20 % steps. The samples A1 and A6 using only one of the crosslinkers have been included in the set for comparison while samples A2 - A5 are samples according to the disclosure. Table 2 lists the respective compositions wherein the amounts are given as the weight in mg.

**Table 2**

| Sample | **A1** | **A2** | **A3** | **A4** | **A5** | **A6** |
|---|---|---|---|---|---|---|
| Ratio short / long [% / %] | 100 / 0 | 80 / 20 | 60 / 40 | 40 / 60 | 20 / 80 | 0 / 100 |
| EGPEA | 11,500 | 11,500 | 11,500 | 11,500 | 11,500 | 11,500 |
| EGDMA | 624 | 499 | 375 | 250 | 125 | 0 |
| TEGMA | 0 | 208 | 416 | 624 | 832 | 1,040 |
| CQ | 30 | 30 | 30 | 30 | 30 | 30 |
| EDMAB | 30 | 30 | 30 | 30 | 30 | 30 |

These sample compositions have been photopolymerized to form buttons for an IOL which were tested for their unfolding time as described above.

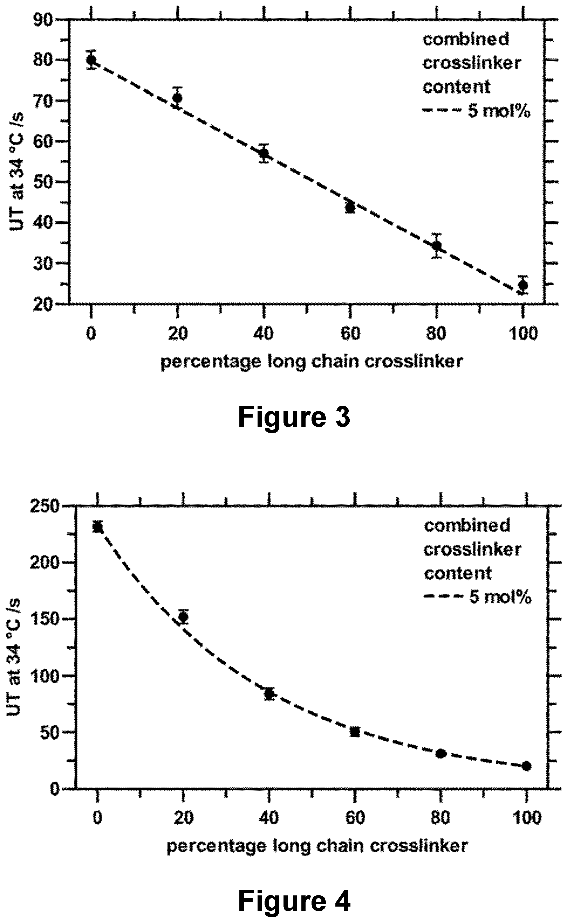

**Figure 3** shows a diagram of the measured unfolding time of these samples. As can be seen, within this pair of EGDMA + TEGDMA, the unfolding time decreases linearly from the pure EGDMA as the shorter crosslinker to the pure TEGDMA as the longer crosslinker.

The results of the mechanical and optical examinations of the buttons are summarized in below Table 3.

**Table 3**

| Sample | **A1** | **A2** | **A3** | **A4** | **A5** | **A6** |
|---|---|---|---|---|---|---|
| Ratio short / long [% / %] | 100 / 0 | 80 / 20 | 60 / 40 | 40 / 60 | 20 / 80 | 0 / 100 |
| T_{g} [°C] | 13 | 13 | 13 | 13 | 13 | 13 |
| RI (589.3 nm, 20 °C) [-] | 1.5546 | 1.5549 | 1.5535 | 1.5525 | 1.5524 | 1.5518 |
| ν (Abbe) [-] | 39.3 | 39.0 | 39.7 | 39.8 | 40.1 | 39.9 |
| IRHD-M [-] | 76.0 | 71.2 | 61.3 | 60.5 | 63.9 | 63.4 |

The T_{g}, RI, and v (Abbe) remain essentially constant over all samples A1 - A6 while the IRHD-M decreases with the content of the short chain crosslinker EGDMA from sample A1 to sample A6. The hardness of the buttons correlates with the unfolding time.

### Example B: BPA-DMA + BPAEO-DMA

The second system of crosslinkers which has been tested used Bisphenol A dimethacrylate (BPA-DMA) and Bisphenol A ethoxylate dimethacrylate (BPAEO-DMA). Hence, the two crosslinkers again differ only in the number of ethylene glycol units (0 vs. 2). When compared to Example A, these crosslinkers differ in a central group (Bisphenol A) with an side chain on each side of different length as opposed to the single intermediate chain of different length. Once more, the ratio of the two crosslinkers which are forming a total amount of 5 mol-% in the composition has been altered in 20 % steps. The samples B1 and B6 using only one of the crosslinkers have been included in the set for comparison while samples B2 - B5 are samples according to the disclosure. Table 4 lists the respective compositions wherein the amounts are given as the weight in mg.

**Table 4**

| Sample | **B1** | **B2** | **B3** | **B4** | **B5** | **B6** |
|---|---|---|---|---|---|---|
| Ratio short / long [% / %] | 100 / 0 | 80 / 20 | 60 / 40 | 40 / 60 | 20 / 80 | 0 / 100 |
| EGPEA | 11,500 | 11,500 | 11,500 | 11,500 | 11,500 | 11,500 |
| BPA-DMA | 1,148 | 918 | 689 | 459 | 230 | 0 |
| BPAEO-DMA | 0 | 340 | 681 | 1,021 | 1,362 | 1,702 |
| CQ | 30 | 30 | 30 | 30 | 30 | 30 |
| EDMAB | 30 | 30 | 30 | 30 | 30 | 30 |

These sample compositions have also been photopolymerized to form buttons for an IOL which were tested for their unfolding time as described above.

**Figure 4** shows a diagram of the measured unfolding time of these samples. As can be seen, within this pair of BPA-DMA + BPAEO-DMA, the unfolding time decreases exponentially from the pure BPA-DMA as the shorter crosslinker to the pure BPAEO-DMA as the longer crosslinker.

The results of the mechanical and optical examinations of the buttons are summarized in below Table 5.

**Table 5**

| Sample | **B1** | **B2** | **B3** | **B4** | **B5** | **B6** |
|---|---|---|---|---|---|---|
| Ratio short / long [% / %] | 100 / 0 | 80 / 20 | 60 / 40 | 40 / 60 | 20 /80 | 0 / 100 |
| T_{g} [°C] | 15 | 15 | 16 | 17 | 16 | 16 |
| RI (589.3 nm, 20 °C) [-] | 1.5648 | 1.5644 | 1.5607 | 1.5612 | 1.5604 | 1.5588 |
| ν (Abbe) [-] | 35.7 | 35.7 | 37.4 | 35.5 | 36.2 | 36.7 |
| IRHD-M [-] | 72.8 | 69.9 | 64.0 | 58.8 | 54.5 | 53.6 |

Again, the T_{g}, RI, and v (Abbe) remain essentially constant over all samples B1 - B6 while the IRHD-M decreases with the content of the short chain crosslinker BPA-DMA from sample B1 to sample B6. Also here, the hardness of the buttons correlates with the unfolding time.

### Comparative test with a single crosslinker

For comparison of the influence on the unfolding time, a sample set C has been prepared with four samples. The set used EGPEA as the polymerizable monomer for the button and 1.25 mol-%, 2.50 mol-%, 3.75 mol-%, and 5.00 mol-%, respectively, of EGDMA as a single crosslinker for samples 1-4. Hence, sample C4 is equal to sample A1.

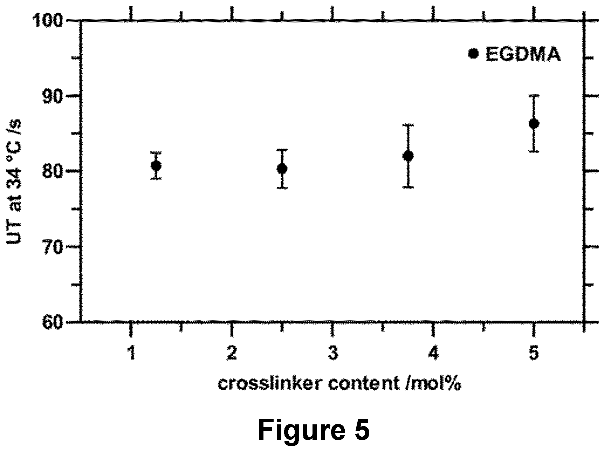

The results of set C are shown in **Figure 5****.** It can be seen that the unfolding time remains essentially constant at about 84 seconds. This demonstrates that it is not possible to adjust the unfolding time of the IOL by using only a single crosslinker and varying its amount. It is essential to use at least two different crosslinkers with different chain lengths.

## Claims

1. A method for making an intraocular lens or a preform thereof comprising
- providing a polymerizable composition comprising
+ at least one polymerizable monomer, oligomer, or polymer,
+ at least a first and a second crosslinker, wherein the first and second crosslinkers have a generic formula A1 - (B)_{q} - A2, with A1, A2 = crosslinkable group and B = backbone group of C, O, Si, and/or S atoms, and wherein first and second crosslinkers differ in a chain length q of the molecules by at least 1 and optionally at most 40, and
+ at least one initiator,
- selecting a molar ratio of first to second crosslinker, and
- polymerizing the composition to form the intraocular lens or a preform thereof.

2. The method of claim 1, comprising
- providing a first polymerizable composition comprising
+ at least one polymerizable monomer, oligomer, or polymer,
+ at least a first and a second crosslinker, wherein the first and second crosslinkers have a generic formula A1 - (B)_{q} - A2, with A1, A2 = crosslinkable group and B = backbone group of C, O, Si, and/or S atoms, and wherein first and second crosslinkers differ in a chain length q of the molecules by at least 1 and optionally at most 40, and
+ at least one initiator,
- selecting a molar ratio R1 of first to second crosslinker, and
- polymerizing the first composition to form a first intraocular lens or a preform thereof; and
- providing a second polymerizable composition comprising
+ the polymerizable monomer, oligomer, or polymer; the first and second crosslinkers; and the initiator,
- selecting a molar ratio R2 of first to second crosslinker, and
- polymerizing the second composition to form a second intraocular lens or a preform thereof.

3. The method according to claim 1 or 2, wherein the method further comprises the steps of
- receiving a desired unfolding time for the intraocular lens, particularly from a medical practitioner, and
- selecting the molar ratio of first to second crosslinker such that the desired unfolding time is obtained.

4. The method according to claim 2 or 3, wherein a total molar amount of crosslinker and/or a molar amount of initiator in the polymerizable composition is held constant while selecting the ratios of first to second crosslinker, in particular the total molar amount of crosslinker and/or molar amount of initiator in the first and second polymerizable compositions are essentially the same.

5. The method according to at least one of claims 2 to 4, wherein a ratio of the longest unfolding time to the shortest unfolding time at 34 °C is at least 2:1, optionally at least 5 : 1 or at least 10 : 1, in particular a ratio of the longest unfolding time to the shortest unfolding time at 34 °C comparing the first and second intraocular lens or preform thereof is at least 2:1, optionally at least 5 : 1 or at least 10:1.

6. The method according to at least one of claims 2 to 5, wherein the unfolding time at 34 °C is adjustable in a range of from 15 s to 300 s and/or is varied with the ratio of the two crosslinkers in a linear or exponential dependency, in particular the unfolding time at 34 °C comparing the first and second intraocular lens or preform thereof is adjustable in a range of from 15 s to 300 s and/or is varied with the ratio of the two crosslinkers in a linear or exponential dependency.

7. The method according to at least one of the preceding claims, wherein selecting the ratio of first to second crosslinker within a given composition changes one or more, optionally all, of the properties
- glass transition temperature T_{g},
- refractive index, determined at 589.3 nm and 20 °C,
- v (Abbe),
- absorption spectrum,
of the intraocular lens or the preform thereof by less than 10 %, optionally by less than 5 %, relative to the lowest one of the compared values.

8. The method according to at least one of the preceding claims, wherein the first and second crosslinkers have a general formula
A1_{B} - spacer_{B1} - (B), - spacer_{B2} -A2_{B}
and
A1_{C} - spacerc, - (C)ₛ - spacer_{C2} -A2_{c},
with A1_{B,C}, A2_{B,C} = crosslinkable group, B, C = backbone group of C, O, Si, and/or S atoms, and spacer_{B1, C1}, spacer_{B2, C2} = 1-10 repeating units, and
wherein first and second crosslinkers differ
i) in a chain length r and s of the molecules by at least 1 and optionally at most 40 and/or
ii) in a number of repeating units in spacer_{B1, C1} and/or spacer_{B2, C2},
and wherein spacer_{B1} ≠ spacer_{C1}, and
wherein one or more, optionally all, of the following definitions apply
a) A1_{B} = A2_{B} = A1_{C} = A2_{C},
b) (B)ᵣ = (C)ₛ,
c) spacer_{B1} = spacer_{B2} and spacer_{C1} = spacer_{C2}.

9. The method according to at least one of the preceding claims, wherein the first and second crosslinkers are
- bifunctional acrylic or methacrylic compounds, optionally α,ω-substituted, and/or
- independently chosen from acrylic or methacrylic compounds having the formula 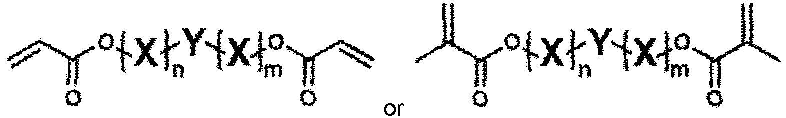
with n, m = 1-10; X = -CH₂-O-, -CH₂-CH₂-O-, -CH₂-CH₂-CH₂-O-, -S-O-, -S-S-O-, or -S-S-S-O-; Y = bisphenol A,
or having the formula 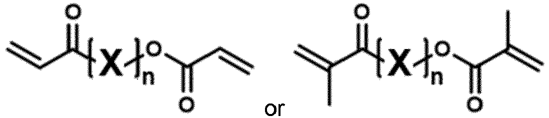
with n = 1-10; X = -CH₂-O-, -CH₂-CH₂-O-, -CH₂-CH₂-CH₂-O-, -S-O-, -S-S-O-, or
-S-S-S-O-.

10. The method according to at least one of the preceding claims, wherein the method comprises the step of making two or more intraocular lenses or preforms thereof, wherein the two or more intraocular lenses or preforms thereof differ from each other in unfolding times, but not in the polymerizable monomers, oligomers, or polymers and/or initiators from which they are made.

11. A composition for intraocular lenses or preforms thereof comprising
- at least one polymerizable monomer, oligomer, or polymer,
- at least a first and a second crosslinker, and
- at least one initiator,
wherein the first and second crosslinkers have a generic formula A1 - (B)_{q} - A2, with A1, A2 = crosslinkable group and B = backbone group of C, O, Si, and/or S atoms, and differ in a chain length q of the molecules by at least 1 and optionally at most 40 and wherein an intraocular lens or preform thereof produced from this composition by polymerization has an unfolding time which is adjustable by selecting a molar ratio of first to second crosslinker.

12. The composition according to claim 11, wherein the first and second crosslinkers are
- bifunctional acrylic or methacrylic compounds, optionally α,ω-substituted, and/or
- independently chosen from acrylic or methacrylic compounds having the formula 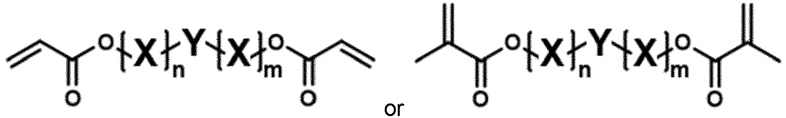
with n, m = 1-10; X = -CH₂-O-, -CH₂-CH₂-O-, -CH₂-CH₂-CH₂-O-, -S-O-, -S-S-O-, or
-S-S-S-O-; Y = bisphenol A,
or having the formula 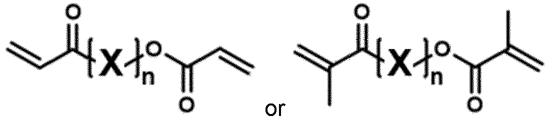
with n = 1-10; X = -CH₂-O-, -CH₂-CH₂-O-, -CH₂-CH₂-CH₂-O-, -S-O-, -S-S-O-, or
-S-S-S-O-.

13. The composition according to at least one of claims 11 or 12, wherein the polymerizable monomer, oligomer, or polymer comprises (meth)acrylates, acrylamides and/or alkylene glycols and/or is selected from the group comprising methyl methacrylate (MMA), 2-hydroxyethyl methacrylate (HEMA), 2-hydroxyethyl acrylate (HEA), butyl acrylate (BA), lauryl acrylate (LA), ethoxyethyl methacrylate (EOEMA), methacrylic acid (MAA), acrylamide (AA), ethylene glycol, propylene glycol, poly(ethylene glycol) phenyl ether acrylate (PEGPEA), and 2-phenylphenoxyethyl acrylate (OPPEA).

14. An article in the form of an ophthalmic medical aid, in particular an intraocular lens or a preform thereof, comprising a polymerized product obtained by polymerizing a composition according to one of claims 11 to 13, and/or by performing the method according to one of claims 1 to 10.

15. The article according to claim 14, wherein the article has an unfolding time at 34 °C in a range of from 15 s to 300 s.
